# FASCICULE DE BREVET EUROPEEN

(11) **EP 1 395 329 B1**
(45) Date de publication et mention de la délivrance du brevet: **12.04.2006**
(21) Numéro de dépôt: 02740839.2
(22) Date de dépôt: 28.05.2002
(51) Int. Cl.: A61M 37/00

(54) **APPAREIL DE TATOUAGE ET DE DERMOPIGMENTATION A USAGE UNIQUE**
VORRICHTUNG ZUM TÄTOWIEREN UND DERMOPIGMENTIEREN FÜR EINMALIGE VERWENDUNGEN
SINGLE-USE TATTOOING AND DERMOPIGMENTATION DEVICE

(30) Priorité: 28.05.2001 FR 0107026
(43) Date de publication de la demande: 10.03.2004
(73) Titulaire: Merkel, Sylvie, 51210 Montmirail (FR)
(72) Inventeur: Merkel, Sylvie, 51210 Montmirail (FR)
(74) Mandataire: Hammond, William
(86) Numéro de dépôt international: PCT/FR2002/001796
(87) Numéro de publication internationale: WO 2002/096499

(56) Documents cités:
- EP-A- 0 374 355
- DE-U- 29 919 199
- FR-A- 2 109 056
- US-A- 4 582 060
- US-A- 4 771 660
- US-A- 5 401 242
- US-A- 5 471 102
- US-A- 5 586 473

## Description

La présente invention est relative à un appareil de tatouage et de dermo-pigmentation à usage unique. Untel appareil comprend traditionnellement, d'une part, un tube cylindrique muni d'un manchon et, d'autre part, une tige porte-aiguille disposée dans ce tube : un exemple de cet appareil est notamment décrit dans le document EP-374 355-A. Un appareil de tatouage selon le préambule de la revendication 1 est décrit par exemple dans FR-A- 21069056.

L'opération de tatouage doit ofrir une totale sécurité sanitaire : c'est pourquoi il est nécessaire de pouvoir le stériliser. De ce fait les appareils de tatouage actuellement sur le marché sont réalisés en un matériau pouvant supporter la stérilisation. Ce matériau est généralement de l'acier inoxydable. Mais néanmoins, le nettoyage et la décontamination d'un tel appareil ne permettent pas d'assurer une totale fiabilité par rapport à la destruction des agents pathogènes tels que notamment le bacille de Koch et le virus HIV.

Par ailleurs, le matériel utilisé présente un autre inconvénient dans la mesure où le centrage des aiguilles pour un travail de précision sans oscillation n'est absolument pas garanti.

Aussi un des buts de la présente invention est-il de fournir un appareil de tatouage et de dermopigmentation à usage unique qui permet de garantir une stérilité totale de celui-ci.

Un autre but de l'invention est de fournir un tel appareil qui permet d'assurer un travail de précision sans oscillation de la tige porte-aiguille dans le tube cylindrique.

Ces buts, ainsi que d'autres qui apparaîtront par la suite, sont atteints par un appareil de tatouage et de dermopigmentation à usage unique comprenant, d'une part, un tube cylindrique muni d'un manchon dont il est solidaire et, d'autre part, une tige porte-aiguille disposée dans ce tube, lequel appareil est caractérisé, selon la présente invention, par le fait que la tige porte-aiguille comporte des moyens d'appui sur la paroi interne du tube cylindrique.

Avantageusement, ces moyens d'appui sont constitués par au moins trois ailettes rayonnant autour de la tige porte-aiguille et régulièrement disposées sur celle-ci.

De préférence, les ailettes ont une largeur telle qu'elles ont pour enveloppe la paroi interne du tube cylindrique.

Avantageusement, les ailettes s'étendent sur une hauteur correspondant sensiblement à celle du manchon.

La description qui va suivre et qui ne comporte aucun caractère limitatif, doit être lue en regard des figures annexées, parmi lesquelles :
- la figure 1 est une vue perspective d'un appareil selon la présente invention ;
- la figure 2 est une vue en plan du tube cylindrique et de la tige porte-aiguille, qui constituent l'appareil représenté à la figure 1 ; et,
- la figure 3 est une vue en coupe de la tige porte-aiguille selon la ligne III-III de la figure 2.

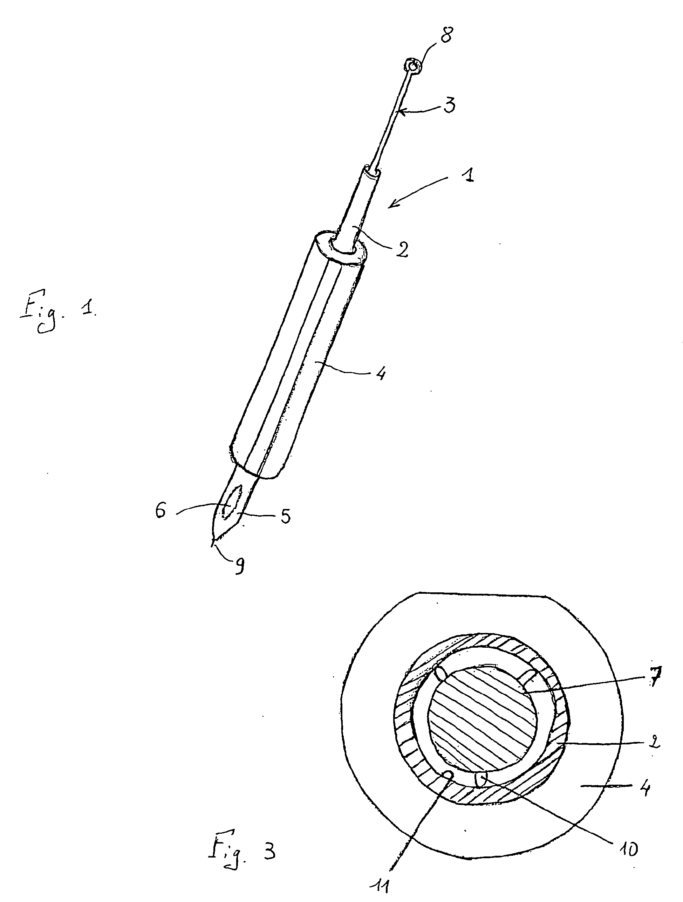

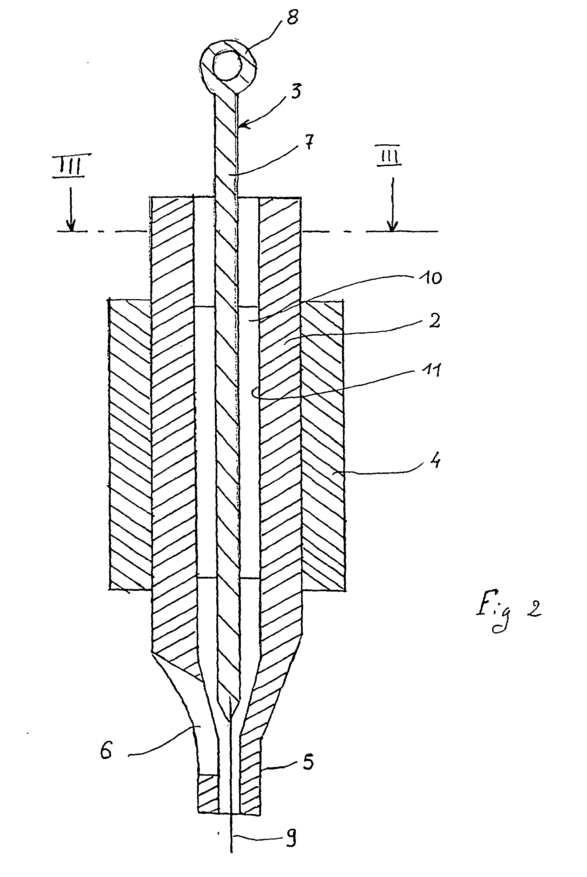

Comme représenté aux figures 1 et 2, un appareil de tatouage et de dermo-pigmentation à usage unique, désigné dans son ensemble par la référence 1, comprend, d'une part, un tube cylindrique 2 et, d'autre part, u ne tige porte-aiguille 3 disposée dans ce tube 2.

Ce tube 2 comporte un manchon 4 de diamètre extérieur standard ou variable qui présente une partie plane pour la préhension, comme cela est connu. De plus, ce manchon 4 est solidaire du tube 2 par tout moyen connu. Il est prolongé à une des ses extrémités par un embout 5 de forme connue selon le groupage des aiguilles. Ce dernier comporte une ouverture 6 pour la capillarité ; celle-ci permet également de visualiser le niveau d'encre.

Quant à la tige porte-aiguille 3, elle est constituée par une tige 7 comprenant, à une extrémité, dite extrémité supérieure, une boucle 8 standard ou une encoche et, à son autre extrémité, dite extrémité inférieure, des moyens connus pour la fixation d'au m oins une aiguille 9. En particulier, cette extrémité inférieure est, selon le groupement d'aiguilles, arrondie ou plate afin de recevoir le faisceau d'aiguilles qui est inséré et collé dans cette partie de la tige 7. Ces aiguilles 9 se trouvent dans l'axe de la tige 7 et ont été stérilisées à la fabrication.

Selon la présente invention, la tige 7 comporte des moyens d'appui 10 sur la paroi interne 11 du tube cylindrique 2. Ces moyens d'appui sont constitués par au moins trois ailettes 10, qui sont régulièrement réparties autour de la tige 7 et ont une largeur telle qu'elles ont pour enveloppe la paroi interne 11 du tube cylindrique 2.

Ces ailettes 10 s'étendent donc sur une hauteur correspondant, au maximum, sensiblement à celle du tube cylindrique 2 : de préférence sur la hauteur du manchon 4.

Lors des opérations de tatouage ou de dermopigmentation, l'appareil selon la présente invention est disposé dans un dispositif connu entraînant la tige 7 dans un mouvement rectiligne de va-et-vient : la tige 7 grâce aux ailettes 10 est maintenue en mouvement selon le même axe, ce qui permet d'assurer le suivi d'un tracé exact par les aiguilles 9 sur la peau d'un patient.

## Revendications

1. Appareil de tatouage et de dermopigmentation à usage unique comprenant, d'une part, un tube cylindrique (2) muni d'un manchon (4) dont il est solidaire et, d'autre part, une tige porte-aiguille (7) disposée dans ledit tube (2) et comportant des moyens d'appui (10) sur la paroi interne (11) dudit tube cylindrique (2), **caractérisé par le fait que** ces moyens d'appui sont constitués par au moins trois ailettes (10) rayonnant autour de la tige porte-aiguille (7) et régulièrement disposées sur celle-ci, ayant une largeur telle qu'elles ont pour enveloppe la paroi interne (11) du tube cylindrique (2) et s'étendant sur une hauteur correspondant sensiblement à celle du manchon (4).

## Patentansprüche

1. Vorrichtung zum Tätowieren und Dermopigmentieren für einmalige Verwendungen, bestehend einerseits aus einem zylindrischen Rohr (2) mit einer darauf fest angeordneten Manschette (4) und andererseits aus einem stiftförmigen Nadelhalter (7), der innerhalb des Rohres (2) angeordnet ist und gegen die Innenwand (11) des Rohres (2) wirkende Abstützmittel aufweist, **dadurch gekennzeichnet, dass** die Abstützmittel von mindestens drei vom Nadelhalter (7) ausgehenden und auf ihm gleichmäßig angeordneten Rippen (10) gebildet sind, wobei die Rippen (10) in ihrer Weite bis zur Innenwand (11) des Rohres (2) reichen und eine Höhe aufweisen, die derjenigen der Manschette (4) in etwa entspricht.

## Claims

1. A single-use tattooing and dermo-pigmentation appliance comprising, on the one hand, a cylindrical tube (2) provided with a sleeve (4) with which it is integral and, on the other hand, a needle-carrying rod (7) arranged in said tube (2) and comprising support means (10) on the internal wall (11) of said cylindrical tube (2), **characterised in that** these support means comprise at least three fins (10) extending outwards round the needle-carrying rod (7) and arranged at regular intervals thereon, having a width such that the internal wall (11) of the cylindrical tube (2) are their envelope and extending over a height corresponding substantially to that of the sleeve (4).
